# EUROPEAN PATENT APPLICATION

(11) **EP 0 561 023 A1**
(43) Date of publication of application: **22.09.1993**
(21) Application number: 92104705.6
(22) Date of filing: 18.03.1992
(51) Int. Cl.: A61F 13/66, A61F 5/44

(54) **Babies' diapers**

(71) Applicant: Machfud, Daniel, Chariae Petach Tikva (IL)
(72) Inventor: Machfud, Daniel, Chariae Petach Tikva (IL)
(74) Representative: König, Beate, Dipl.-Phys. Dr.

(57) **Abstract**

This invention relates to a diaper for absorbing the secretion of urine and feces comprising of a vinyl or rubber layer (1) to prevent the leakage of wastes with protruding sides around its circumference and with a hollowed tube (2) along the sides for impenetrability and softness of touch with the baby's body, of a contracted elastic tube located at the narrow part of the diaper on both sides for maximum proximity to the baby's body at the fold of the diaper, of an absorbers (7) of urine and an absorber (7) of feces located on top of the vinyl or rubber layer (1) between the sides, of a partition (4) to the width of the narrow part of the diaper dividing between the urine absorber and the feces absorber, and of adhesive strips (5,6) to close the diaper around the body.

## Description

The present invention relates to babies diapers. More specifically the invention relates to a new and economical diaper wherein only the absorbent part of the diaper is changed.

Today we are familiar with cloth diapers that can be reused many times as well as paper diapers that are discarded after each use, and also paper diapers for one-time usage made of an absorbent layer, plastic covering and adhesive tape. The main disadvantage of the multi-usage cloth diaper is that the diaper needs thorough washing after each secretion of feces or urine, entailing much work and expenses on cleaning materials. The single usage diapers save the work involved in laundry, cleaning and laundry expenses as well as cleaning materials but they too have various disadvantages, to whit:
a. High purchasing expenses when calculated for long term use. A baby uses about 5-6 diapers each day. Thus the expenses on 6,000 single-usage diapers over 3 years could be very significant.
b. The bother - it is necessary to carry home large packages of paper diapers.
c. Storage of diapers necessitates a large volume of space both at home and in stores.
d. Throwing the single usage paper diapers to the garbage creates ecological problems.
e. Each secretion, whether urine or feces, entails changing the paper diaper.
f. The diaper is not hermetically sealed, and therefore sometimes the moisture seeps over onto the baby clothing and bedding.

The present invention aims to overcome all these disadvantages, both of multi usage cloth diapers and single usage paper diapers.

The present invention relates to a diaper absorbing secretions either of feces or urine comprised of a vinyl or rubber component to prevent the seepage of the secretions with protruding sides (about a half a centimeter high) around its circumference and with a hollowed tube along the sides for impenetrability and softness in touching the baby's body, and of a contracted elastic tube positioned at the narrower part of the diaper on both sides for maximum proximity at the fold of the diaper to the baby's body, an absorber of urine and an absorber of feces positioned upon the vinyl or rubber component between the sides, of a partition positioned at the width of the narrow part of the diaper between the urine absorber and the feces absorber, and of adhesive stripes to close the diaper around the body.

The vinyl or rubber layer is shaped similarly to the conventional diaper, with two triangles joined at their vertex. In the said diaper the vertex is the narrow part between the baby's legs, whereas one "triangle" is the front part covering the stomach and absorbing the urine, and the second "triangle" is at the rear of the body absorbing the feces. The vinyl or rubber layer is surrounded by walls wherein the absorbent layers are located on the vinyl or rubber layer between the walls. The said invention has two absorbent layers in each diaper, one absorbent layer at the front triangle for the purpose of absorbing urine and a second absorbent layer at the rear triangle aimed at absorbing feces. There is a partition between the two absorbent layers, comprised of a partition made of vinyl or rubber or any other similar material. This partition can be an integral part of the vinyl or rubber. After the baby has secreted wastes, it is possible to replace only the soiled absorbent layer and put a new absorbent layer in the appropriate triangle within the vinyl or rubber layer. There is no need to replace the entire diaper and certainly not the non-soiled absorbent layer. If, for example, the baby had no secretion of feces it is necessary to replace only one absorbent layer out of the two.

When the relevant absorbent layer is for one-time use, the baby's parents have to purchase only absorbents and not whole diapers. It is cheaper and more convenient, both for storage purposes and with regard to carrying the diapers from the store. When the relevant absorbent layer is made of cloth for multi usage it saves the money used for repurchasing diapers and adds convenience of changing as compared with conventional diapers made of cloth.

The greatest advantage of the said invention is that it is not necessary to replace the entire diaper in case of urine secretion (or feces), but only to replace the appropriate absorbent layer.

The diaper according to the invention is attached hermetically to the body with no possibility of leakage onto clothing or bedding due to the transparent plastic tube surrounding the vinyl or rubber layer on the walls. This tube both creates impenetrability and also is soft to the touch on the baby's skin. A contracted elastic tube is located at the narrow part of the diaper on both sides for maximum attachment to the baby's body at the fold of the diaper.

The absorbent layer can be made of any absorbing material. The absorbent layers can be used once or several times.

A multi-usage absorbent layer would be made of cloth and could be used repeatedly after washing. An absorbent layer can be made for example, of cotton with 8 layers wherein the two upper layers extend the diaper's dimensions by 10 mm and the remaining six layers are of the same size as the diaper.

A one-time absorbent layer can be made of paper, cotton or any other combination thereof.

The diaper according to the present invention can be used both by babies and adults suffering from incontinence. The preferred fasteners of the diapers are adhesive tapes, but it is possible to use any fastener of any kind capable of joining together the front part and the rear part of the diaper.

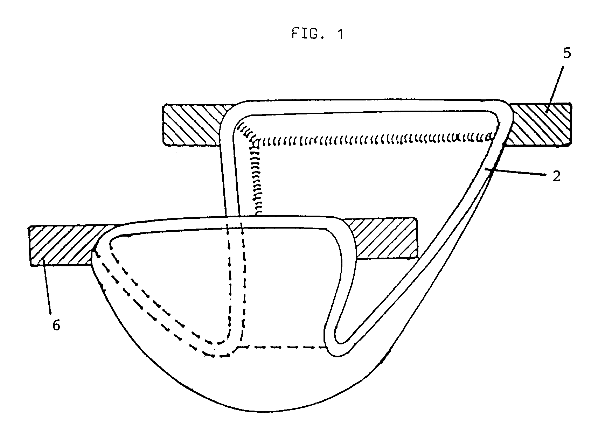

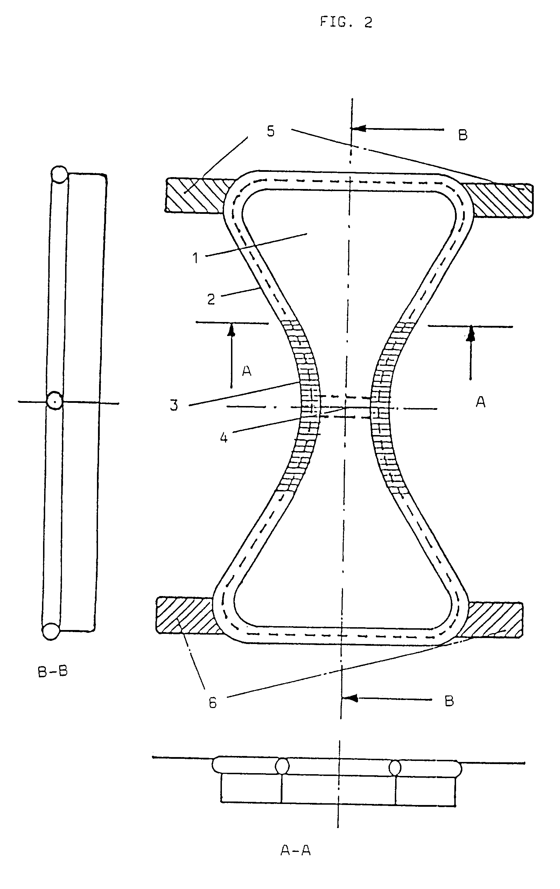

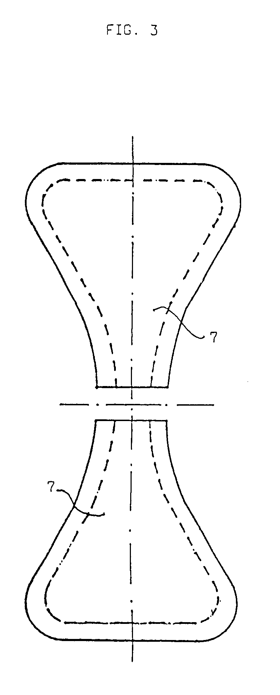

The invention will be exemplified and clarified by means of Figures 1, 2 and 3. These figures are in no way intended to limit the scope of the invention, and serve only as illustrations.

Figure 1 describes isometrically the diaper with adhesive tapes.

Figure 2 describes the diaper and its parts in a view from above: The vinyl or rubber layer (1) is surrounded by the hollowed tube (2) a contracted elastic tube in its center (3) the partition (4) divides the vinyl or rubber layer into two triangle parts with slightly rounded corners. The adhesive strips (5) and (6) will be used when the diaper is put on in order to attach it to the baby's body.

Figure 3 describes the absorbent layers (7) that are to be placed on the vinyl or rubber layer on both sides of the partition.

## Claims

1. A diaper for absorbing the secretion of urine and feces comprising of a vinyl or rubber layer (1) to prevent the leakage of wastes with protruding sides around its circumference and with a hollowed tube (2) along the sides for impenetrability and softness of touch with the baby's body, of a contracted elastic tube located at the narrow part of the diaper on both sides for maximum proximity to the baby's body at the fold of the diaper, of an absorber (7) of urine and an absorber (7) of feces located on top of the vinyl or rubber layer (1) between the sides, of a partition (4) to the width of the narrow part of the diaper dividing between the urine absorber and the feces absorber, and of adhesive strips (5, 6) to close the diaper around the body.

2. A diaper according to claim 1 wherein the partition (4) is an integral part of the vinyl or rubber layer (1).

3. A diaper according to claim 1 wherein the layer for absorbing urine and/or feces is made of cloth such that it can be reused after laundry.

4. A diaper according to claim 3 wherein the absorbent layer is made of eight layers of cotton wherein the two upper layers extend beyond the diaper by 10 mm and the six remaining layers are of the same size as the diaper.

5. A diaper according to claim 1 wherein the absorbing layer of the feces and/or of the urine is a single-usage absorbent layer made of paper, cotton or any combination of the two materials, or any other absorbing material conventionally used in the diaper industry.
